(19) Europäisches Patentamt
European Patent Office
Office européen des brevets

(11) Numéro de publication: **0 406 042 B1**

(12) # FASCICULE DE BREVET EUROPEEN

(45) Date de publication de fascicule du brevet: **04.01.95**

(51) Int. Cl.6: **A61K 7/06**, A61K 7/48

(21) Numéro de dépôt: **90401575.7**

(22) Date de dépôt: **08.06.90**

(54) **Composition cosmétiques contenant comme agent épaississant un polymère ayant une faible proportion de motifs à groupements ioniques.**

(30) Priorité: **09.06.89 LU 87534**

(43) Date de publication de la demande:
**02.01.91 Bulletin 91/01**

(45) Mention de la délivrance du brevet:
**04.01.95 Bulletin 95/01**

(84) Etats contractants désignés:
**AT BE CH DE DK ES FR GB GR IT LI NL SE**

(56) Documents cités:
**FR-A- 2 238 474**
**FR-A- 2 305 967**
**FR-A- 2 345 144**
**US-A- 3 972 336**

(73) Titulaire: **L'OREAL**
**14, Rue Royale**
**F-75008 Paris (FR)**

(72) Inventeur: **Mondet, Jean**
**34 rue Daniel Fery**
**F-93700 Drancy (FR)**
Inventeur: **Papantoniou, Christos**
**17, rue des Basserons**
**F-95160 Montmorency (FR)**
Inventeur: **Vanlerberghe, Guy**
**Rue du Général de Gaulle**
**F-77000 Montigny la Tour (FR)**

(74) Mandataire: **Tonnellier, Jean-Claude et al**
**Cabinet Nony & Cie.**
**29, rue Cambacérès**
**F-75008 Paris (FR)**

**EP 0 406 042 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des compositions cosmétiques ou supports de compositions cosmétiques sous la forme d'une émulsion du type eau-dans-l'huile, contenant comme agent épaississant au moins un copolymère comportant une faible proportion de motifs contenant des groupements ioniques.

On sait que les compositions cosmétiques sous forme d'émulsions du type eau-dans-l'huile se présentent sous la forme de laits ou de crèmes. Les compositions sous forme de laits sont des émulsions fluides. Les crèmes sont des émulsions épaisses de viscosité généralement supérieure à 1,5 Pa.s.

La préparation des crèmes sous forme d'émulsions du type eau-dans-l'huile nécessite l'épaississement de la phase continue huileuse de l'émulsion. Généralement, l'épaississement est obtenu en incorporant une cire dans la phase huileuse. Toutefois, les crèmes épaissies à l'aide de cires ont un toucher généralement considéré comme désagréable.

La présente invention a pour objet de préparer des crèmes épaissies du type eau-dans-l'huile qui ne présentent pas cet inconvénient. Dans les crèmes de l'invention, l'épaississement est obtenu à l'aide de copolymères contenant une faible proportion de groupements ioniques ou ionisables.

L'invention a donc pour objet une composition cosmétique ou un support de composition cosmétique sous la forme d'une émulsion épaissie du type eau-dans-l'huile, caractérisée par le fait qu'elle contient un agent épaississant constitué par au moins un copolymère comprenant :

des motifs de formule I

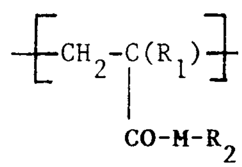

(I)

où M est un atome d'oxygène ou un groupement $-N(R_3)-$,

$R_1$ représente -H ou $-CH_3$,

$R_2$ est une chaîne hydrocarbonée saturée, linéaire ou ramifiée, ayant 4 à 22 atomes de carbone,

$R_3$ représente -H ou une chaîne hydrocarbonée saturée, linéaire ou ramifiée, ayant 1 à 22 atomes de carbone,

et des motifs de formule II

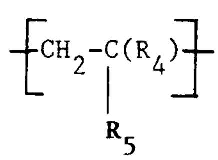

(II)

dans laquelle :

soit $R_4$ représente -H ou $-CH_3$,

et $R_5$ représente alors un groupement

-CO-X-Z-Y,

X représente un atome d'oxygène ou le groupement $-N(R_6)-$

$R_6$ représente soit H, soit une chaîne hydrocarbonée saturée ayant 1 à 22 atomes de carbone,

Z représente une chaîne hydrocarbonée saturée linéaire ou ramifiée ayant 2 à 22 atomes de carbone, éventuellement interrompue par des groupements -NH- ou par des hétéroatomes -O-,

Y représente -COOH ou $-SO_3H$,

soit $R_4$ représente -H et $R_5$ représente $-SO_3H$, $-CH_2SO_3H$ ou $-C_6H_4-SO_3H$,

étant entendu que ledit copolymère contient, en motifs, de 90 à 99% de motifs de formule I, les autres motifs étant constitués par les motifs de formule II, que lesdits groupements -COOH, $-SO_3H$, $-CH_2SO_3H$ et $-C_6H_4SO_3H$ présents sont sous forme partiellement ou totalement salifiée, et étant entendu que lorsque $R_5$ représente $-C_6H_4-SO_3H$, M représente un groupement $-N(R_3)-$.

Parmi les copolymères utilisables comme agents épaississants selon l'invention, on peut distinguer en particulier: ceux qui ne contiennent pas de groupement $-SO_3H$, libres ou salifiés ; ceux qui ne contiennent pas de groupement -COOH, libres ou salifiés.

Les copolymères utilisés peuvent aussi contenir à la fois des groupements $-SO_3H$,libres ou salifiés, et des groupements -COOH, libres ou salifiés.

Les copolymères utilisés comme agents épaississants selon la présente invention peuvent être obtenus par copolymérisation de monomères de formule III

$CH_2 = C(R_1)-CO-M-R_2$ (III)

dans laquelle $R_1$, M et $R_2$ sont définis comme précédemment, avec des monomères de formule IV

$CH_2 = C(R_4)-R_5$ (IV)

dans laquelle $R_4$ et $R_5$ sont définis comme précédemment.

Il est également possible de préparer certains copolymères de l'invention par copolymérisation de styrène et de monomères de formule III bis

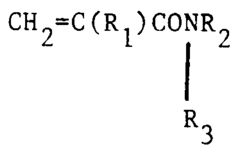

(IIIbis)

dans laquelle $R_1$, $R_2$ et $R_3$ sont définis comme précédemment, pour obtenir un copolymère correspondant que l'on soumet à l'action d'un agent de sulfonation pour obtenir un copolymère dont les motifs comportant des groupements ioniques répondent à la formule V

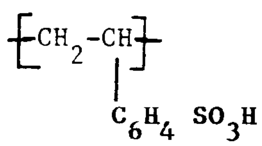

(V)

La réaction de copolymérisation est effectuée selon les techniques usuelles, par exemple en solution, en suspension ou en émulsion.

La masse moléculaire des copolymères varie généralement de 5.000 à 500.000 environ.

Les monomères de formules III, IIIbis et IV sont des produits connus ou peuvent être préparés selon les méthodes connues.

Dans des modes de réalisation particuliers de l'invention, les copolymères utilisés comme agents épaississants peuvent encore présenter les caractéristiques suivantes, prises isolément ou en combinaison :

- $R_2$ représente un groupement alkyle ayant de 4 à 18 atomes de carbone ;
- $R_3$ est H ou un groupement alkyle ayant 1 à 18 atomes de carbone ;
- Z représente un groupement alkylène ayant 2 à 22 atomes de carbone.

Parmi les monomères qui sont à l'origine des motifs de formule I on peut citer par exemple les acrylates et méthacrylates d'hexyle, d'éthyl-2 hexyle, de dodécyle et d'octadécyle ainsi que les acrylamides et méthacrylamides correspondants (avec M = NH au lieu de O). On peut également citer les N-éthyl N-dodécyl (et les N-butyl N-dodécyl) acrylamides et méthacrylamides.

Parmi les monomères conduisant aux motifs de formule II, on peut citer par exemple l'acide acrylamido-2 méthyl-2 propane sulfonique, l'(acrylamido-10 méthyl-10) décanoate de 2-sulfoéthyle, le méthacrylate de 2-sulfoéthyle, l'acide vinyl sulfonique et l'acide allyl sulfonique.

Dans les copolymères utilisés selon l'invention, les motifs de formule II doivent être au moins en partie présents sous forme de sels, la salification pouvant être totale. Les sels sont notamment des sels métalliques tels que des sels de métaux alcalins, des sels de métaux alcalino-terreux, des sels de zinc, ou encore des sels d'amines tels que des sels de triéthanolamine, des sels d'amino-2 méthyl-2 propanol-1,

des sels d'amino-2 méthyl-2 propanediol-1,3, etc... Parmi les sels métalliques on peut citer notamment les sels de lithium, de potassium, de sodium, de zinc, de calcium, de magnésium, etc...

Dans les compositions cosmétiques ou supports de compositions cosmétiques selon l'invention, la teneur en copolymères épaississants tels que définis ci-dessus est généralement de 0,05 à 5% en poids, et de préférence de 0,1 à 2% en poids.

En raison de la présence majoritaire des motifs de formule I dans les copolymères épaississants utilisés selon l'invention, ces copolymères n'ont pas de caractère hydrophile et sont solubles dans les huiles.

Lorsque le copolymère n'est pas suffisamment soluble dans la phase huileuse pour atteindre la concentration désirée, dans la gamme qui vient d'être indiquée, il est possible de favoriser la solubilisation du copolymère à l'aide d'un co-solvant organique compatible avec l'utilisation en cosmétologie. Parmi les co-solvants on peut citer par exemple un alcool tel que l'éthanol, le propanol, l'isopropanol, le glycérol, le propylène glycol, etc... Le co-solvant est ajouté dans la phase huileuse avant mélange avec la phase aqueuse de l'émulsion.

Généralement la proportion de co-solvant, lorsqu'il est présent, n'est pas supérieure à 30% en volume, par rapport au volume de la phase huileuse.

La phase huileuse est constituée d'huiles ou de mélanges d'huiles habituellement utilisées dans les cosmétiques. Ces huiles et leurs propriétés sont décrites dans la littérature spécialisée. Ce sont notamment des triglycérides, des silicones, des hydrocarbures, etc...

Parmi les huiles utilisables on peut citer par exemple celles qui sont mentionnées dans la partie expérimentale ci-après.

Les copolymères utilisés comme agents épaississants selon l'invention présentent des propriétés d'agents tensio-actifs. Lorsque les propriétés tensio-actives sont insuffisantes pour permettre l'obtention d'une émulsion stable, il est nécessaire de prévoir, dans la composition ou le support de composition cosmétique, la présence d'un agent émulsionnant usuel, en quantité suffisante pour que l'émulsion soit stable.

Les agents émulsionnants compatibles avec l'utilisation en cosmétologique sont connus et décrits dans la littérature spécialisée.

Parmi les agents émulsionnant utilisables, on citera à titre d'exemple non limitatif l'isostéarate de glycérol vendu par Dynamit Nobel sous la dénomination IMWITOR 780K, et les éthers de polyglycérols répondant à la formule suivante :

$$C_{12}H_{25}\text{-}CH(C_{10}H_{21})\text{-}CH_2O\{C_2H_3O(CH_2OH)\}_nH$$

avec n = 2 à 15 (valeur statistique), qui sont décrits dans la demande de brevet français 2.593.509.

Généralement, la proportion d'agent émulsionnant, lorsqu'il est présent, peut aller jusqu'à 15% en poids par rapport au poids total de la composition cosmétique ou du support de composition cosmétique.

Les compositions cosmétiques selon l'invention peuvent contenir les ingrédients usuels présents dans ces compositions (ingrédients actifs, parfums, agents conservateurs, filtres solaires, etc...).

Ces ingrédients peuvent être ajoutés, selon les techniques connues, soit avant, soit pendant, soit après la réalisation de l'émulsion.

Les compositions cosmétiques contenant les émulsions épaissies du type eau-dans-l'huile obtenues selon l'invention sont d'un aspect agréable. Leur texture est légère et contrairement aux émulsions classiques de ce type, elles ne sont pas trop grasses, notomment au toucher. Par ailleurs, elles pénètrent bien, et elles laissent à l'application une impression de fraîcheur.

Elles peuvent être utilisées pour le soin et l'hydratation du visage et du corps, et également comme produit de soin pour les mains.

Les supports de composition cosmétique selon l'invention peuvent aussi servir de base, par exemple, pour la réalisation des compositions de protection contre le rayonnement solaire.

L'invention a également pour objet l'utilisation, comme agent épaississant, dans la préparation de compositions cosmétiques ou de supports de compositions cosmétiques sous la forme d'émulsions du type eau-dans-l'huile, d'au moins un copolymère tel que défini ci-dessus.

L'invention a en outre pour objet un procédé de préparation d'une composition cosmétique ou d'un support de composition cosmétique, sous la forme d'une émulsion épaissie du type eau-dans-l'huile, ce procédé étant caractérisé par le fait que l'on incorpore dans la composition ou le support de composition, comme agent épaississant, au moins un copolymère tel que défini précédemment. De préférence, on incorpore l'agent épaississant dans la phase huileuse, éventuellement à l'aide d'un co-solvant comme indiqué précédemment, avant la réalisation de l'émulsion. On ajoute si désiré un agent émulsionnant usuel

pour favoriser la stabilité de l'émulsion, comme indiqué ci-dessus.

Les exemples suivants illustrent l'invention sans toutefois la limiter.

EXEMPLES DE PREPARATION

1. Copolymère N-dodécylacrylamide (96,5% en poids)/Acide acrylamido-2 méthyl-2 propane sulfonique (A.M.P.S.) (3,5% en poids) ; sel de sodium

Dans un réacteur avec agitation mécanique et barbotage d'azote, on introduit 96,5g de N-dodécylacrylamide et 240g de toluène. On dissout sous agitation à 35 °C.

Parallèlement, on réalise une solution de 3,5g d'A.M.P.S. dans 60g de méthanol, en dissolvant sous agitation à 30 °C.

La solution méthanolique maintenue à 30 °C est introduite dans le réacteur maintenu sous agitation à 35 °C. Les deux solutions se mélangent immédiatement en donnant un milieu limpide.

On introduit 1g d'initiateur azobisisobutyronitrile et chauffe le milieu réactionnel sous agitation jusqu'au reflux (66 °C).

On maintient ces conditions pendant 8 heures, puis laisse refroidir à température ambiante.

La solution de polymérisation est concentrée à l'évaporateur rotatif d'abord sous une pression de l'ordre de 20mm de mercure (on rappelle qu'à 1mm de mercure correspond une pression d'environ 130 Pa) et à 35 °C jusqu'à l'apparition d'un gel (distillation de 70g de solvant).

On continue l'évaporation sous une pression d'environ 5mm de mercure à 35 °C jusqu 'à obtention d'un résidu de 160g correspondant à un mélange polymère-solvant à l'aspect solide.

On ajoute alors 640g de tétrahydrofuranne et on agite jusqu'à dissolution à température ambiante.

Une fois la solution obtenue on effectue un prélèvement pour analyse. Le prélèvement est précipité dans l'eau, puis séché sous vide à température ambiante.

On fait un dosage volumétrique des motifs acide dans un mélange toluène/méthanol (95:5 en volume) en utilisant de la soude (solution méthanolique 0,1N) et un mélange alizarine/thymol phtaléine comme indicateur coloré. On trouve 15 méq. d'acide/100g de polymère.

Neutralisation par la soude

La solution de polymère dans le tétrahydrofuranne est neutralisée sous agitation à température ambiante par la quantité stoechiométrique d'hydroxyde de sodium (en solution méthanolique 1N) correspondant à une neutralisation totale des motifs acide sulfonique.

La solution neutralisée est concentrée à l'évaporateur rotatif à 35 °C sous une pression de 20mm de mercure jusqu'à un poids de 300g.

La solution concentrée est précipitée dans 4 litres d'eau permutée refroidie à 10 °C.

Le précipité obtenu est séché sous vide à 50 °C jusqu'à poids constant.

Poids obtenu : 84g.

| Analyse élémentaire sur le polymère neutralisé : | | | | | | |
|---|---|---|---|---|---|---|
| Elément | C% | H% | N% | O% | S% | Na% |
| Théorie | 73,82 | 11,86 | 5,86 | 7,51 | 0,54 | 0,39 |
| Trouvé | 72,36 | 11,85 | 5,69 | 8,94 | 0,50 | 0,48 |

EXEMPLE 2

Copolymère N-tertiobutylacrylamide (97,9% en poids)-Acide acrylamido-2 méthyl-2 propane sulfonique (2,1% en poids) ; sel de sodium.

Dans un reacteur avec agitation mécanique et barbotage d'azote on introduit 97,9g de N-tertiobutylacrylamide et 210g d'éthanol absolu. On agite à température ambiante jusqu'à dissolution.

Parallèlement on réalise une solution de 2,1g d'acide acrylamido-2 méthyl-2 propane sulfonique (A.M.P.S.) dans 90g d'eau permutée.

La solution aqueuse est introduite dans le réacteur sous agitation. On ajoute 1g d'initiateur azobisisobutyronitrile.

On chauffe pour obtenir une température intérieure de 70 °C. On maintient sous agitation à 70 °C pendant 8 heures.

On refroidit ensuite le milieu, en ajoutant 70g d'éthanol absolu lorsque la température est descendue à 55 °C, pour éviter la précipitation du polymere.

La solution reste ensuite limpide jusqu'à température ambiante.

Avant neutralisation on effectue un prélèvement de la solution que l'on précipite dans de l'eau froide. On sèche le précipité sous vide à température ambiante. Indice d'acide en utilisant la méthode décrite dans l'exemple 1 : 5 méq./100g de polymère.

La solution de polymère est neutralisée sous agitation à température ambiante par ajout de la quantité stoechiométrique de soude aqueuse 1N correspondant à une neutralisation totale. On laisse réagir pendant une heure.

La solution neutralisée est précipitée dans 5 litres d'eau permutée additionnée de glace. On sèche le précipité en étuve à 50 °C sous vide.

Poids obtenu : 90g

| Analyse élémentaire sur le polymère acide (avant neutralisation): | | | | | |
|---|---|---|---|---|---|
| Elément | C% | H% | N% | O% | S% |
| Théorie | 65,60 | 10,15 | 10,93 | 12,99 | 0,33 |
| Trouvé | 63,62 | 10,17 | 10,63 | 15,28 | 0,33 |

EXEMPLE 3

Copolymère N-tertio butylacrylamide (98% en poids)/acide acrylamido 3-méthyl 3-butanoïque (2% en poids) ; sel de sodium et sel de triéthanolamine

Dans un réacteur avec agitation mécanique et barbotage d'azote on introduit 2g d'acide acrylamido 3-méthyl 3-butanoïque, 98g de N-tertio butylacrylamide, 90g d'éthanol absolu, 10g d'eau permutée et 0,25g d'initiateur azobisisobutyronitrile. On porte sous agitation et barbotage d'azote à 55 °C. On laisse réagir dans ces conditions 19 heures à 55 °C. Après retour à la température ambiante, on dilue le mélange réactionnel par un mélange constitué de 180g d'éthanol et 80g d'eau, puis on verse dans 4 litres d'eau glacée.

On sèche le précipité sous vide à 50 °C.

Poids obtenu : 99,7g - Rendement : 99,7%

Indice d'acide par potentiométrie : 14,7 méq./100g polymère.

La neutralisation finale est faite in situ dans le mélange d'huile que l'on veut émulsionner, en introduisant soit la quantité stoechiométrique de soude (solution méthanolique), soit la quantité stoechiométrique de triéthanolamine.

EXEMPLE 4

Copolymère méthacrylate de stéaryle (92,2% en poids) - Méthacrylate de 2-sulfoéthyle (7,8% en poids) ; sel de sodium.

Dans un réacteur avec agitation mécanique et barbotage d'azote on introduit 46,1g de méthacrylate de stéaryle et 70g de toluène.

On obtient une solution limpide en agitant à température ambiante.

Parallèlement on dissout sous agitation à température ambiante 3,9g de méthacrylate de 2-sulfoéthyle dans 40g d'éthanol.

La solution éthanolique est introduite dans le réacteur. On mélange sous agitation pour obtenir un milieu homogène. On introduit 0,75g d'initiateur azobisisobutyronitrile.

On chauffe sous agitation et barbotage d'azote à 70 °C.

On ajoute aussi 23,5g de toluène dans le milieu réactionnel, par portions, pendant les trois premières heures de réaction, pour que le milieu reste homogène.

On laisse réagir 12 heures au total à 70 ° C.

Lorsque l'on ramène le milieu à température ambiante en fin de réaction on doit ajouter à nouveau du toluène en quantité suffisante pour éviter la précipitation du polymère formé dans le réacteur.

Un prélèvement de la solution de polymérisation est précipité dans du méthanol pour analyse.

On sèche le prélèvement sous vide à température ambiante. Indice d'acide suivant la méthode décrite à l'exemple 1 : 31,4 méq./100 g de polymère.

Neutralisation de la solution de polymérisation par la soude

On ajoute goutte à goutte sous agitation et à température ambiante la quantité stoechiométrique de soude (solution méthanolique 1N) correspondant à une neutralisation totale, en diluant le milieu si nécessaire par du toluène pour éviter la précipitation du polymère.

On purifie le polymère neutralisé par précipitation dans 3 litres de méthanol refroidi à 10 ° C, puis séchage sous vide à 50 ° C.

Poids obtenu : 26,7 g.

EXEMPLE 5

Synthèse d'un copolymère N-dodécylacrylamide-styrène sulfonate de zinc

1) Copolymérisation dodécylacrylamide/styrène (95%mole/5% mole).

Dans un réacteur avec agitation centrale et barbotage d'azote on introduit 293,3g de dodécylacrylamide, 6,7g de styrène, 150g d'acétate d'éthyle et 0,75g d'initiateur azobisisobutyronitrile. On dissout sous agitation et barbotage d'azote. On chauffe à 77 ° C, et effectue la polymérisation à cette température sous agitation et barbotage d'azote pendant 20 heures.

Après retour à température ambiante, on purifie le polymère obtenu par précipitation dans 5 litre de méthanol, puis séchage du précipité sous vide à 50 ° C.

Poids obtenu : 292,5g (rendement : 97,5%)

2) Sulfonation des motifs styrène.

100g du polymère précédent sont dissous dans 600g de dichloro-1,2 éthane sous agitation. La dissolution totale nécessite plusieurs heures.

On chauffe à 53 ° C sous barbotage d'azote et on introduit successivement 2730 microlitres d'anhydride acétique puis 1000 microlitres d'acide sulfurique à 95%.

On laisse réagir sous agitation à 50 ° C et sous barbotage d'azote. On ajoute ensuite 50g d'éthanol absolu dans le milieu à 50 ° C pour arrêter la réaction. On refroidit à température ambiante.

On verse goutte à goutte la solution de sulfonation dans 4 litres d'eau bouillante (distillation flash). Le précipité blanc est recueilli sous forme d'une masse compacte, qui est soumise à un broyage en présence de 1 litre d'eau permutée. Le polymère se présente alors sous forme de grains compacts, que l'on sèche par lyophilisation.

Poids obtenu : 97g.

Indice d'acide suivant la méthode décrite dans l'exemple 1 : 9 méq./100g de polymère.

3) Neutralisation totale des motifs acide sulfonique par l'acétate de zinc

On dissout 40g du polymère acide du stade précédent dans 360ml de toluène. On ajoute 40ml de n-propanol.

La solution obtenue est chauffée à 90 ° C sous barbotage d'azote.

On ajoute alors 1,37g d'acétate de zinc (dihydrate).

On laisse réagir sous agitation à 90 ° C pendant 3 heures.

La solution finale est refroidie et précipitée dans 5 litres de méthanol.

On sèche à 50 ° C sous vide.

Poids obtenu : 35,7g.

EXEMPLE 6

Synthèse d'un copolymère N-octadécylacrylamide (96,5% en poids)/acide acrylamido-2 méthyl-2 propane sulfonique (A.M.P.S.) (3,5% en poids) ; sel de sodium.

Dans un reacteur avec agitation mécanique et barbotage d'azote on introduit 44,2g de N-octadécyl acrylamide et 110g de toluène.

On dissout sous agitation à température ambiante.

Parallèlement, on réalise une solution à température ambiante de 1,6g d'acide acrylamido-2 méthyl-2 propane sulfonique (A.M.P.S.) dans 27,5g de méthanol.

La solution méthanolique est introduite dans le réacteur.

On mélange sous agitation et barbotage d'azote.

On introduit 0,458g d'initiateur azobisisobutyronitrile.

On chauffe à 66 °C sous agitation et barbotage d'azote.

On polymérise dans ces conditions pendant 12 heures.

La solution de polymère est concentrée au maximum par évaporation sous vide suivant les conditions de l'exemple 1.

On dissout le résidu dans 105g de tétrahydrofuranne à température ambiante.

Un prélèvement est précipité dans le méthanol pour analyse et séché sous vide à température ambiante.

Indice d'acide sur le prélevement suivant le dosage utilisé dans l'exemple 1 : 14,5 méq./100g de polymère.

| Analyse élémentaire sur le prélèvement : | | | | | |
|---|---|---|---|---|---|
| Elément | C% | H% | N% | O% | S% |
| Théorie | 76,71 | 12,47 | 4,42 | 5,86 | 0,54 |
| Trouvé | 74,95 | 12,79 | 4,35 | 7,40 | 0,49 |

Neutralisation totale par la soude : 150g de solution dans le tétrahydrofuranne sont neutralisés sous agitation à température ambiante par la quantité de soude (solution méthanolique 1N) correspondant à une neutralisation totale.

On laisse réagir une heure sous agitation.

Le sel de sodium est purifié par précipitation dans 2 litres de méthanol, puis séchage sous vide à 50 °C jusqu'à poids constant.

Poids obtenu : 32,5g

EXEMPLES D'UTILISATION

Réalisation et étude d'émulsions E/H épaissies avec les polymères selon l'invention

Les émulsions sont toutes réalisées suivant les mêmes conditions.

Le polymère provoquant l'épaississement de l'émulsion est dissous dans l'huile ou le mélange d'huiles choisi en présence de l'émulsionnant et de la quantité d'alcool nécessaire à la mise en solution (éthanol, isopropanol, propylèneglycol etc...) .

La dissolution est faite à température ambiante sous agitation ou plus facilement en chauffant sous agitation sans évaporation de l'alcool.

La phase aqueuse contient de préférence du sulfate de magnésium $MgSO_4$, $7H_2O$ pour obtenir une meilleure stabilité.

L'émulsion est réalisée à température ambiante en utilisant un disperseur Moritz (marque commerciale) agité à 3.000 tours/minute avec introduction progressive de la phase aqueuse dans la phase organique. L'émulsion peut également être réalisée à température plus élevée (inférieure à 100 °C) avec préchauffage des deux phases. On utilisera alors de préférence le propylène glycol pour dissoudre le polymère dans la phase organique.

La viscosité de l'émulsion est mesurée au viscosimètre Contraves (marque commerciale) à 25 °C.

Les données sur les constituants des émulsions et sur les viscosités obtenues sont résumées dans le Tableau I qui suit.

Notations abrégées

ET : éthanol ; IP : isopropanol ; PG : propylèneglycol ;

PIB : polyisobutylène hydrogéné vendu par la Société NICHIYA sous la marque PARLEAM ; HP : huile de paraffine ; HT : huile de tournesol; HK : huile de Karité ; MIP : myristate d'isopropyle ; FNS : benzoate d'alkyle en $C_{12}$-$C_{15}$ commercialisé par WITCO sous la marque FINSOLV TN ; CPDS : cyclopenta diméthyl siloxane, huile de silicone commercialisée par UNION CARBIDE ; IMW : glycéride partiellement modifié par l'acide isostéarique, commercialisé par DYNAMIT NOBEL sous la marque IMWITOR-780K ; EPG : éther de polyglycérol de formule

$$C_{12}H_{25}\text{-}CH(C_{10}H_{21})\text{-}CH_2O\text{-}[C_2H_3O(CH_2OH)]_n H$$

avec n = 3 (valeur statistique).

Toutes les émulsions ont été préparées à température ambiante, sauf dans le cas de l'exemple C (80 ° C).

Le polymère utilisé à l'exemple K est celui de l'exemple 3 (1g) neutralisé in situ avec la triéthanolamine (TEA : 22mg).

TABLEAU I

| EXEMPLE | POLYMERE DE L'EXEMPLE N° | COSOLVANT | PHASE HUILEUSE | EMULSIONNANT | EAU | SULFATE DE MAGNESIUM | VISCOSITE Pa.s. |
|---|---|---|---|---|---|---|---|
| A | Ex.1 : 1g | IP : 1ml | PIB : 28g | | 70g | | 4 |
| B | Ex.1 : 1g | ET : 1ml | PIB : 16g | IMW : 5g | 76,5g | 0,5g | 10 |
| C | Ex.1 : 0,5g | PG : 0,1ml | PIB : 16g | IMW : 5g | 76,5g | 2g | 7 |
| D | Ex.1 : 1g | ET : 1ml | PIB : 20g | IMW : 5g | 71g | 2g | 4,8 |
| E | Ex.1 : 0,5g | ET : 0,5ml | CPDS : 5g<br>HP : 12g | IMW : 5g | 75g | 2g | 6,5 |
| F | Ex.1 : 0,2g | ET : 0,2ml | FNS : 25g | IMW : 5g | 67,6g | 2g | 6,5 |
| G | Ex.1 : 1g | ET : 1ml | HT : 40g | IMW : 5g | 51g | 2g | 6 |
| H | Ex.1 : 0,2g | ET : 0,2ml | HK : 45g | IMW : 5g | 47,6g | 2g | 7,3 |
| I | Ex.1 : 2g | ET : 2ml | PIB : 18g | EPG : 3g | 74g | 1g | 7,3 |
| J | Ex.2 : 1g | ET : 1ml | HP : 18g | IMW : 5g | 73g | 2g | 7 |
| K | Ex.3 : 1,022 g | ET : 1ml | MIP : 20g | IMW : 5g | 71g | 2g | 3,1 |
| L | Ex.4 : 2g | ET : 0,2ml | PIB : 48g | | 50g | | 2 |
| M | Ex.5 : 1g | IP : 3ml | HT : 28g | | 68,6g | | 2,8 |
| N | Ex.6 : 1g | | PIB : 20g | IMW : 5g | 72g | 2g | 7,5 |

## Revendications

1. Composition cosmétique ou support de composition cosmétique sous la forme d'une émulsion épaissie du type eau-dans-l'huile, caractérisée par le fait qu'elle contient un agent épaississant constitué par un copolymère comprenant :

   des motifs de formule I

$$\left[ CH_2-C(R_1) \right] \quad \text{avec } CO-M-R_2 \text{ sur } C \qquad (I)$$

où M est un atome d'oxygène ou un groupement $-N(R_3)-$,

$R_1$ représente -H ou $-CH_3$,

$R_2$ est une chaîne hydrocarbonée saturée, linéaire ou ramifiée, ayant 4 à 22 atomes de carbone,

$R_3$ représente -H ou une chaîne hydrocarbonée saturée, linéaire ou ramifiée, ayant 1 à 22 atomes de carbone, et des motifs de formule II

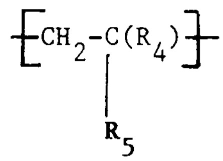

(II)

dans laquelle :

soit $R_4$ représente -H ou $-CH_3$,

et $R_5$ représente alors un groupement

-CO-X-Z-Y,

X représente un atome d'oxygène ou le groupement $-N(R_6)-$

$R_6$ représente soit H, soit une chaîne hydrocarbonée saturée ayant 1 à 22 atomes de carbone,

Z représente une chaîne hydrocarbonée saturée linéaire ou ramifiée ayant 2 à 22 atomes de carbone, éventuellement interrompue par des groupements -NH- ou par des hétéroatomes -O-,

Y représente -COOH ou $-SO_3H$,

soit $R_4$ représente -H et $R_5$ représente $-SO_3H$, $-CH_2SO_3H$ ou $-C_6H_4-SO_3H$,

étant entendu que ledit copolymère contient, en motifs, de 90 à 99% de motifs de formule I, les autres motifs étant constitués par les motifs de formule II, que lesdits groupements -COOH, $-SO_3H$, $-CH_2SO_3H$ et $C_6H_4SO_3H$ présents sont sous forme partiellement ou totalement salifiée et étant entendu que lorsque $R_5$ représente $-C_6H_4-SO_3H$, M représente un groupement $-N(R_3)-$.

**2.** Composition ou support selon la revendication 1, caractérisée par le fait que ledit copolymère ne contient pas de groupements $-SO_3H$, libres ou salifiés.

**3.** Composition ou support selon la revendication 1, caractérisée par le fait que ledit copolymère ne contient pas de groupements -COOH, libres ou salifiés.

**4.** Composition ou support selon la revendication 1, caractérisée par le fait que les motifs de formule I sont dérivés d'au moins un des monomères suivants : acrylates et méthacrylates d'hexyle, d'éthyl-2 hexyle, de dodécyle et d'octadécyle, acrylamides et méthacrylamides correspondants (avec M = NH au lieu de O), N-éthyl N-dodécyl acrylamide ou méthacrylamide, et N-butyl N-dodécyl acrylamide ou méthacrylamide.

**5.** Composition ou support selon l'une quelconque des revendications précédentes, caractérisée par le fait que les motifs de formule II sont dérivés d'au moins un des monomères suivants : acide acrylamido-2 méthyl-2 propane sulfonique, (acrylamido-10 méthyl-10) décanoate de 2-sulfoéthyle, méthacrylate de 2-sulfoéthyle, acide vinyl sulfonique et acide allyl sulfonique.

**6.** Composition ou support selon l'une quelconque des revendications précédentes, caractérisée par le fait que la masse moléculaire du copolymère est comprise entre 5.000 et 500.000.

**7.** Composition ou support selon l'une quelconque des revendications précédentes, caractérisée par le fait que lesdits groupements -COOH et/ou $-SO_3H$ sont salifiés sous forme de sels métalliques ou sous forme de sels d'amine.

**8.** Compositions ou support selon la revendication 7, caractérisées par le fait que lesdits sels métalliques sont des sels de métaux alcalins, de métaux alcalino-terreux ou de zinc.

**9.** Composition ou support selon la revendication 8, caractérisée par le fait que lesdits sels sont des sels de lithium, de potassium, de sodium, de zinc, de calcium ou de magnésium.

**10.** Composition ou support selon la revendication 7, caractérisée par le fait que lesdits sels d'amines sont des sels de triéthanolamine, d'amino-2 méthyl-2 propanol-1 ou d'amino-2 méthyl-2 propanediol-1,3.

**11.** Composition ou support selon l'une quelconque des revendications précédentes, caractérisée par le fait que la teneur en copolymère est de 0,05 à 5% en poids.

**12.** Composition ou support selon la revendication 7, caractérisée par le fait que la teneur en copolymère est de 0,1 à 2% en poids.

**13.** Composition ou support selon l'une quelconque des revendications précédentes, caractérisée par le fait que la phase huileuse contient un co-solvant favorisant la solubilisation dudit copolymère.

**14.** Composition ou support selon la revendication 13, caractérisée par le fait que ledit co-solvant est un alcool choisi parmi l'éthanol, le propanol, l'isopropanol, le glycérol et le propylène glycol.

**15.** Composition selon l'une quelconque des revendications 13 et 14, caractérisée par le fait que la proportion de co-solvant n'est pas supérieure à 30% en volume, par rapport au volume de la phase huileuse.

**16.** Utilisation d'un copolymère tel que défini dans l'une quelconque des revendications 1 à 10, comme agent épaississant dans la préparation d'une composition cosmétique ou d'un support de composition cosmétique sous la forme d'une émulsion du type eau-dans-l'huile.

**17.** Utilisation selon la revendication 16, caractérisée par le fait que ladite composition cosmétique ou ledit support de composition cosmétique est telle que définie dans l'une quelconque des revendications 11 à 15.

**18.** Procédé de préparation d'une composition cosmétique ou d'un support de composition cosmétique sous la forme d'une émulsion épaissie du type eau-dans-l'huile, caractérisé par le fait que l'on incorpore dans ladite composition ou dans ledit support, comme agent épaississant, au moins un copolymère tel que défini dans l'une quelconque des revendications 1 à 10.

**19.** Procédé selon la revendication 18, caractérisé par le fait que l'on incorpore l'agent épaississant dans la phase huileuse, éventuellement à l'aide d'un co-solvant, avant la réalisation de l'émulsion.

**20.** Procédé selon la revendication 18 ou 19, caractérisé par le fait que ladite composition présente les caractéristiques définies dans au moins l'une quelconque des revendications 11 à 15.

**Claims**

**1.** Cosmetic composition or cosmetic composition vehicle in the form of a thickened emulsion of water-in-oil type, characterized in that it contains a thickening agent consisting of a copolymer comprising:

units of formula I

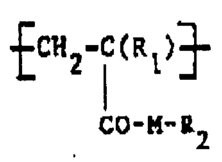

(I)

where M is an oxygen atom or an -N($R_3$)- group,
$R_1$ represents -H or -$CH_3$,
$R_2$ is a linear or branched saturated hydrocarbon chain having 4 to 22 carbon atoms,
$R_3$ represents -H or a linear or branched saturated hydrocarbon chain having 1 to 22 carbon atoms,
and units of formula II

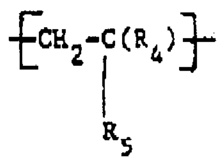

(II)

in which:

either $R_4$ represents -H or $-CH_3$,

and $R_5$ then represents a -CO-X-Z-Y group,

X represents an oxygen atom or the $-N(R_6)-$ group,

$R_6$ represents either H or a saturated hydrocarbon chain having 1 to 22 carbon atoms,

Z represents a linear or branched saturated hydrocarbon chain having 2 to 22 carbon atoms, optionally interrupted by -NH- groups or by -O- heteroatoms,

Y represents -COOH or $-SO_3H$,

or $R_4$ represents -H and $R_5$ represents $-SO_3H$, $-CH_2SO_3H$ or $-C_6H_4-SO_3H$,

it being understood that the said copolymer contains, as units, from 90 to 99% of units of formula I, the other units consisting of the units of formula II, that the said -COOH, $-SO_3H$, $-CH_2SO_3H$ and $C_6H_4SO_3H$ groups present are in the partially or entirely salified form and it being understood that when $R_5$ represents $-C_6H_4-SO_3H$, M represents an $-N(R_3)-$ group.

**2.** Composition or vehicle according to Claim 1, characterized in that the said copolymer does not contain free or salified $-SO_3H$ groups.

**3.** Composition or vehicle according to Claim 1, characterized in that the said copolymer does not contain free or salified -COOH groups.

**4.** Composition or vehicle according to claim 1, characterized in that the units of formula I are derived from at least one of the following monomers: hexyl, 2-ethylhexyl, dodecyl and octadecyl acrylates and methacrylates, corresponding acrylamides and methacrylamides (with M = NH in place of O), N-ethyl-N-dodecylacrylamide or N-ethyl-N-dodecylmethacrylamide, and N-butyl-N-dodecylacrylamide or N-butyl-N-dodecylmethacrylamide.

**5.** Composition or vehicle according to any one of the preceding claims, characterized in that the units of formula II are derived from at least one of the following monomers: 2-acrylamido-2-methylpropanesulphonic acid, 2-sulphoethyl 10-acrylamido-10-methyldecanoate, 2-sulphoethyl methacrylate, vinylsulphonic acid and allylsulphonic acid.

**6.** Composition or vehicle according to any one of the preceding claims, characterized in that the molecular mass of the copolymer is between 5,000 and 500,000.

**7.** Composition or vehicle according to any one of the preceding claims, characterized in that the said -COOH and/or $-SO_3H$ groups are salified in the form of metal salts or in the form of amine salts.

**8.** Composition or vehicle according to Claim 7, characterized in that the said metal salts are alkali metal, alkaline-earth metal or zinc salts.

**9.** Composition or vehicle according to Claim 8, characterized in that the said salts are lithium, potassium, sodium, zinc, calcium or magnesium salts.

**10.** Composition or vehicle according to Claim 7, characterized in that the said amine salts are triethanolamine, 2-amino-2-methyl-1-propanol or 2-amino-2-methyl-1,3-propanediol salts.

**11.** Composition or vehicle according to any one of the preceding claims, characterized in that the copolymer content is from 0.05 to 5% by weight.

**12.** Composition or vehicle according to Claim 7, characterized in that the copolymer content is from 0.1 to 2% by weight.

**13.** Composition or vehicle according to any one of the preceding claims, characterized in that the oily phase contains a cosolvent which promotes solubilization of the said copolymer.

**14.** Composition or vehicle according to Claim 13, characterized in that the said cosolvent is an alcohol chosen from ethanol, propanol, isopropanol, glycerol and propylene glycol.

**15.** Composition or vehicle according to either one of Claims 13 and 14, characterized in that the proportion of cosolvent is not greater than 30% by volume with respect to the volume of the oily phase.

**16.** Use of a copolymer such as defined in any one of Claims 1 to 10 as thickening agent in the preparation of a cosmetic composition or of a cosmetic composition vehicle in the form of an emulsion of water-in-oil type.

**17.** Use according to Claim 16, characterized in that the said cosmetic composition or the said cosmetic composition vehicle is as defined in any one of Claims 11 to 15.

**18.** Process for the preparation of a cosmetic composition or of a cosmetic composition vehicle in the form of a thickened emulsion of water-in-oil type, characterized in that at least one copolymer as defined in any one of Claims 1 to 10 is incorporated in the said composition or in the said vehicle as thickening agent.

**19.** Process according to Claim 18, characterized in that the thickening agent is incorporated in the oily phase, optionally with the help of a cosolvent, before the preparation of the emulsion.

**20.** Process according to Claim 18 or 19, characterized in that the said composition has the characteristics defined in at least any one of Claims 11 to 15.

**Patentansprüche**

**1.** Kosmetische Zubereitung oder Träger für eine kosmetische Zubereitung in Form einer fertigen Emulsion vom Typ Wasser-in-Öl, dadurch gekennzeichnet, daß sie ein Verdickungsmittel enthält, welches aus einem Copolymer zusammengesetzt ist, das folgende Einheiten umfaßt:
Einheiten gemäß Formel I:

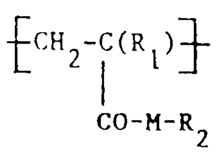

(I),

worin M ein Sauerstoffatom oder die Gruppierung $-N(R_3)-$,
$R_1$ -H oder $-CH_3$,
$R_2$ eine gesättigte lineare oder verzweigte Kohlenwasserstoffkette mit 4 bis 22 Kohlenstoffatomen,
$R_3$ -H oder eine gesättigte lineare oder verzweigte Kohlenwasserstoffkette mit 1 bis 22 Kohlenstoffatomen bedeuten,
sowie Einheiten gemäß der Formel II:

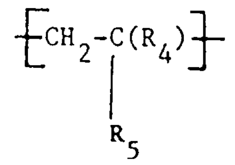

(II),

worin,

$R_4$ Wasserstoff oder $-CH_3$ darstellt,

wobei $R_5$ in dem Falle die Gruppierung -CO-X-Z-Y bedeutet, wenn

X ein Sauerstoffatom oder die Gruppierung $-N(R_6)-$ darstellt,

$R_6$ entweder Wasserstoff oder eine gesättigte Kohlenwasserstoffkette mit 1 bis 22 Kohlenstoffatomen bedeutet,

Z eine gesättigte lineare oder verzweigte Kohlenwasserstoffkette mit 2 bis 22 Kohlenstoffatomen bedeutet, welche gegebenenfalls durch -NH-Gruppierungen oder durch -O- Heteroatome unterbrochen ist,

Y -COOH oder $-SO_3H$ darstellt, oder

$R_4$ Wasserstoff und $R_5$ $-SO_3H$, $-CH_2SO_3H$ oder $-C_6H_4-SO_3H$bedeuten,

und zwar unter der Voraussetzung, daß das Copolymer im Hinblick auf die Einheiten durch 90 bis 99 % Einheiten gemäß Formel I, und die restlichen Einheiten durch die Einheiten gemäß Formel II gebildet werden, wobei die genannten Gruppen -COOH, $-SO_3H$, $-CH_2SO_3H$ sowie $C_6H_4SO_3H$ teilweise oder vollständig in Salzform vorliegen, sowie die weitere Maßgabe gilt, daß dann wenn $R_5$ $-C_6H_4-SO_3H$ bedeutet, M eine $-N(R_3)$-Gruppierung bedeutet.

**2.** Zubereitung oder Träger gemäß Anspruch 1, dadurch gekennzeichnet, daß das Copolymer keine $-SO_3H$-Gruppen in freier oder Salzform enthält.

**3.** Zubereitung oder Träger gemäß Anspruch 1, dadurch gekennzeichnet, daß das Copolymer keine -COOH-Gruppen in freier oder Salzform enthält.

**4.** Zubereitung oder Träger gemäß Anspruch 1, dadurch gekennzeichnet, daß die Einheiten gemaß Formel I mindestens von einem der folgenden Monomere abgeleitet sind: von Hexyl-, Hexyl-2-Ethyl-, Dodecyl- und Octadecylacrylaten und -Methacrylaten, den entsprechenden Acrylamiden und Methacrylamiden (mit M = NH anstelle von O), N-Ethyl-N-Dodecylacrylamid oder -methacrylamid sowie N-Butyl-N-Dodecylacrylamid oder -methacrylamid.

**5.** Zubereitung oder Träger gemäß einem der vorstehenden Ansprüche, dadurch gekennzeichnet, daß die Einheiten gemäß Formel I von mindestens einem der folgenden Monomere abgeleitet sind: 2-Acrylamid-2-Methylpropansulfonsäure, (10-Acrylamid-10-Methyl)-2-Sulfoethyldecanoat, 2-Sulfoethylmethacrylat, Vinylsulfonsäure sowie Allylsulfonsäure.

**6.** Zubereitung oder Träger gemäß einem der vorstehenden Ansprüche, dadurch gekennzeichnet, daß das molekulare Massengewicht des Copolymeren eine Zahl zwischen 5 000 und 500 000 umfaßt.

**7.** Zubereitung oder Träger gemäß einem der vorstehenden Ansprüche, dadurch gekennzeichnet, daß die -COOH- und/oder $-SO_3H$-Gruppen in Salzform als Metallsalze oder in Form der Aminsalze vorliegen.

**8.** Zubereitungen oder Träger gemäß Anspruch 7, dadurch gekennzeichnet, daß die Metallsalze als Alkalimetallsalze, Erdalkalimetallsalze oder Zinksalze vorliegen.

**9.** Zubereitung oder Träger gemäß Anspruch 8, dadurch gekennzeichnet, daß die Salze als Lithiumsalze, Kaliumsalze, Natriumsalze, Zinksalze, Kalzium- oder Magnesiumsalze vorliegen.

**10.** Zubereitung oder Träger gemäß Anspruch 7, dadurch gekennzeichnet, daß die Aminsalze als Triethanolamin-, 2-Amino-2-Methyl-1-Propanol- oder 2-Amino-2-Methyl-1,3-Propandiolsalze vorliegen.

**11.** Zubereitung oder Träger gemäß einem der vorstehenden Ansprüche, dadurch gekennzeichnet, daß der Gehalt an Copolymer zwischen 0,05 und 5 Gew.-% beträgt.

**12.** Zubereitung oder Träger gemäß Anspruch 7, dadurch gekennzeichnet, daß der Copolymergehalt zwischen 0,1 und 2 Gew.-% beträgt.

**13.** Zubereitung oder Träger gemäß einem der vorstehenden Ansprüche, dadurch gekennzeichnet, daß die ölige Phase ein zusätzliches Lösungsmittel enthält, welches die Solubilisierung des Copolymeren begünstigt.

**14.** Zubereitung oder Träger gemäß Anspruch 13, dadurch gekennzeichnet, daß das zusätzliche Lösungsmittel durch einem Alkohol gebildet wird, welcher unter Ethanol, Propanol, Isopropanol, Glycerin und Propylenglykol ausgewählt ist.

**15.** Zubereitung gemäß einem der Ansprüche 13 und 14, dadurch gekennzeichnet, daß der Anteil an zusätzlichem Lösungsmittel 30 Vol.-% in Bezug auf das Volumen der öligen Phase nicht übersteigt.

**16.** Verwendung eines Copolymeren gemäß Definition nach einem der Ansprüche 1 bis 10 als Verdickungsmittel bei der Herstellung einer kosmetischen Zubereitung oder eines Trägers einer kosmetischen Zubereitung in Form einer Emulsion vom Typ Wasser-in-Öl.

**17.** Verwendung gemäß Anspruch 16, dadurch gekennzeichnet, daß die kosmetische Zubereitung oder der Träger für die kosmetische Zubereitung gemäß einem der Ansprüche 11 bis 15 definiert ist.

**18.** Verfahren zur Herstellung einer kosmetischen Zubereitung oder eines Trägers für eine kosmetische Zubereitung in Form einer verdickten Emulsion vom Typ Wasser-in-Öl, dadurch gekennzeichnet, daß man in diese Zubereitung oder in den Träger als Verdickungsmittel mindestens ein Copolymer einarbeitet, wie es gemäß einem der vorstehenden Ansprüche 1 bis 16 definiert ist.

**19.** Verfahren gemäß Anspruch 18, dadurch gekennzeichnet, daß das Verdickungsmittel in die Ölphase, gegebenenfalls mit Hilfe eines zusätzlichen Lösungsmittels, vor dem Aufbereiten zu einer Emulsion eingearbeitet wird.

**20.** Verfahren gemäß Anspruch 18 oder 19, dadurch gekennzeichnet, daß die Zubereitung die in mindestens einem der Ansprüche 11 bis 15 definierten Merkmale aufweist.